# EUROPÄISCHE PATENTSCHRIFT

(11) **EP 0 551 794 B1**
(45) Veröffentlichungstag und Bekanntmachung des Hinweises auf die Patenterteilung: **24.01.1996**
(21) Anmeldenummer: 92810988.3
(22) Anmeldetag: 11.12.1992
(51) Int. Cl.: A61F 2/34

(54) **Zweiteilige Hüftgelenkpfanne**
Two-part acetabular cup
Cupule acétabulaire en deux parties

(30) Priorität: 16.01.1992 CH 112/92
(43) Veröffentlichungstag der Anmeldung: 21.07.1993
(73) Patentinhaber: SULZER Medizinaltechnik AG, CH-8404 Winterthur (CH); ALLO PRO AG, CH-6340 Baar (CH)
(72) Erfinder: Hauser, René, Dr. med., CH-8702 Zollikon (CH); Sandoz, Yvan, CH-8405 Winterthur (CH)
(74) Vertreter: Hammer, Bruno, Dr.

(56) Entgegenhaltungen:
- EP-A- 0 359 700
- EP-A- 0 402 810
- DE-A- 3 205 527
- FR-A- 2 633 509

## Beschreibung

Die Erfindung betrifft eine zweiteilige Hüftgelenkspfanne zur Verankerung im Beckenknochen, bestehend aus einer kegelstumpfförmigen Hülse und einem die eigentliche Pfannenschale enthaltenden, im Passitz in den Innenhohlraum der Hülse einpressbaren Einsatz, wobei die aus Metall gefertigte Hülse in Umfangsrichtung Bereiche grösserer Wandstärke alternierend mit solchen geringerer Wandstärke aufweist, wobei ferner die Bereiche geringerer Wandstärke im Querschnitt Kreisringsektoren sind, während die Bereiche grösserer Wandstärke einen im wesentlichen trapezförmigen Querschnitt haben, der sich zum Pol hin konisch verjüngt.

Eine Hüftgelenkspfanne der vorstehend genannten Art ist bekannt aus der EP-A-0 359 700; muss diese bekannte Pfanne bei Reoperationen ersetzt werden, so ergibt sich für den Operateur sehr häufig die Schwierigkeit, dass für eine stabile Verankerung der "Ersatz"-Pfanne in der Umgebung des operativ geschaffenen Pfannenhohlraumes im Becken nicht mehr genügend Knochensubstanz vorhanden ist.

Aufgabe der vorliegenden Erfindung ist es, die genannte bekannte Pfanne so zu erweitern, dass einerseits ihre an sich bewährte Grundkonzeption auch beim geschilderten Fehlen an Knochensubstanz im Becken beibehalten werden kann und andererseits eine feste Verankerung der "Ersatz"-Pfanne erreicht wird. Selbstverständlich ist die Erfindung dabei nicht auf Reoperationsfälle beschränkt, sondern immer anwendbar, wenn die für eine Pfannenverankerung notwendige Knochensubstanz im Becken fehlt.

Mit der vorliegenden Erfindung wird diese Aufgabe dadurch gelöst, dass die Bereiche grösserer Wandstärke als sich entlang des Mantel des Kegelstumpfes erstreckende Gleitschuhe für aussen aufschiebbare Stützkörper ausgebildet sind, und dass jeder dieser Bereiche mit Fixierungselementen für diese Stützkörper versehen ist.

Jeder Hülse wird dabei ein Bausatz verschiedener Stützkörper zugeordnet, die unterschiedliche Abmessungen - z.B. unterschiedliche Dicken - und/oder unterschiedliche Form - z.B. in der Dicke vom basisseitigen Ende aus stetig oder stufenförmig zu- oder abnehmend - haben. Aus diesem Bausatz kann der Operateur dann einen oder mehrere Stützkörper für den individuellen Fall passend auswählen, die ausgewählten Stützkörper auf die Hülse aufschieben und mit Hilfe der Fixierungselemente an ihr fixieren.

Als Verbindung zwischen Hülse und Stützkörper hat es sich bewährt, wenn die Stützkörper den Trapezquerschnitt der Bereiche grösserer Wandstärke umgreifende, schwalbenschwanzähnliche Ausnehmungen haben und vom Pol her auf die Gleitschuhe aufschiebbar sind.

Die Bereiche grösserer Wandstärke durchsetzende Stiftschrauben mit kegelförmiger Zentrierspitze sind beispielsweise eine geeignete Art der Fixierungselemente; greifen die Stiftschrauben in an ihre Zentrierspitze angepasste, hohlkegelförmige Ausnehmungen der Stützkörper ein, so wird neben der Fixierung gleichzeitig eine genaue Positionierung des Stützkörpers relativ zur Hülse erreicht.

Zur zusätzlichen Abstützung der Pfanne auf dem Beckenknochen kann mindestens ein Teil der Stützkörper an ihrem der Basis des Kegelstumpfes zugewandten Ende mit nach aussen weisenden Laschen versehen sein, die darüberhinaus zur besseren Fixierung der Pfanne Durchtrittsbohrungen für Knochenschrauben haben können.

Ein Anwachsen von Knochengewebe bzw. die Haftung zwischen Knochenzement und Hülse bzw. Stützkörper können schliesslich gefördert werden, wenn die Bereiche grösserer Wandstärke der Hülse und/oder die Stützkörper auf ihrer äusseren Mantelfläche mit einer Querverzahnung in Umfangsrichtung versehen sind.

Im folgenden wird die Erfindung anhand von Ausführungsbeispielen im Zusammenhang mit der Zeichnung näher erläutert.
- Fig. 1: Zeigt in räumlicher Darstellung eine Hülse als Grundkörper, auf dem zwei Stützkörper fixiert sind;
- Fig. 2a bis 2c: gibt eine erste Ausführungsform eines Stützkörpers wieder, wobei 2a eine Seitenansicht ist, 2b einen Schnitt B-B von 2a darstellt, und 2c eine Aufsicht auf die der Basis des Kegelstumpfes zugeordnete Stirnfläche von 2a in Richtung des gezeigten Pfeiles C wiedergibt;
- Fig. 3a und 3b, sowie 4a und 4b: sind zwei weitere Beispiele für unterschiedliche Stützkörper, wobei 3a und 4a die Schnitte A-A von 3b bzw. 4b sind, während diese beiden Teilfiguren der Figur 2c entsprechende Aufsichten darstellen;
- Fig. 5a und 5b: sind den Figuren 2a und 2c entsprechende Darstellungen eines weiteren Stützkörpers;
- Fig. 6: schliesslich zeigt ein vergrössertes Beispiel einer als Fixierungselement dienenden Stiftschraube.

Die aus Metall, beispielsweise aus Titan oder einer Titanlegierung, gefertigte Hülse 1 ist in ihrer Grundform ein Kegelstumpf und besteht in Umfangsrichtung alternierend aus Bereichen 3 grösserer Wandstärke D und Bereichen 4 geringerer Wandstärke d, die Kreisringsektoren mit parallele Wänden bilden. Weiterhin weisen die Bereiche 4 von beiden Stirnseiten des Kegelstumpfes her Ausnehmungen 10 auf, durch die ihre Flexibilität erhöht wird.

Wie in dem gezeigten Ausführungsbeispiel einer Hülse 1 kann diese darüberhinaus längs einer Mantellinie über ihre ganze Höhe geschlitzt sein, was die Flexibilität und Elastizität der Hülse 1 ebenfalls vergrössert.

Darüberhinaus sind die Bereiche 3 grösserer Wandstärke D aussen durch eine Querverzahnung 5 in Umfangsrichtung strukturiert, was das An- und Einwachsen von Knochengewebe fördert.

Die Bereiche 3 grösserer Wandstärke D sind im Querschnitt trapezförmig und von der Kegelstumpfbasis zum Pol hin konisch zulaufend, wodurch sie Gleitschuhe für sie schwalbenschwanz-ähnlich umgreifende Stützkörper 11 bilden, die vom Pol her auf die Bereiche 3 aufschiebbar sind. Die Stützkörper 11 sind dabei mit Vorteil in ihrer Länge längs einer Mantellinie derart an die Hülse 1 angepasst, dass die Stirnseiten beider bei montiertem Stützkörper 11 mindestens annähernd bündig sind.

Jeder Bereich 3 ist in Radialrichtung von einer Gewindebohrung 12 durchsetzt, in die eine mit einem Gewinde 14 versehene Stiftschraube 13 (Fig. 6) als Fixierungs- und gleichzeitig als Zentrierelement für einen Stützkörper 11 von der Innenseite der Hülse 1 her eingeschraubt wird.

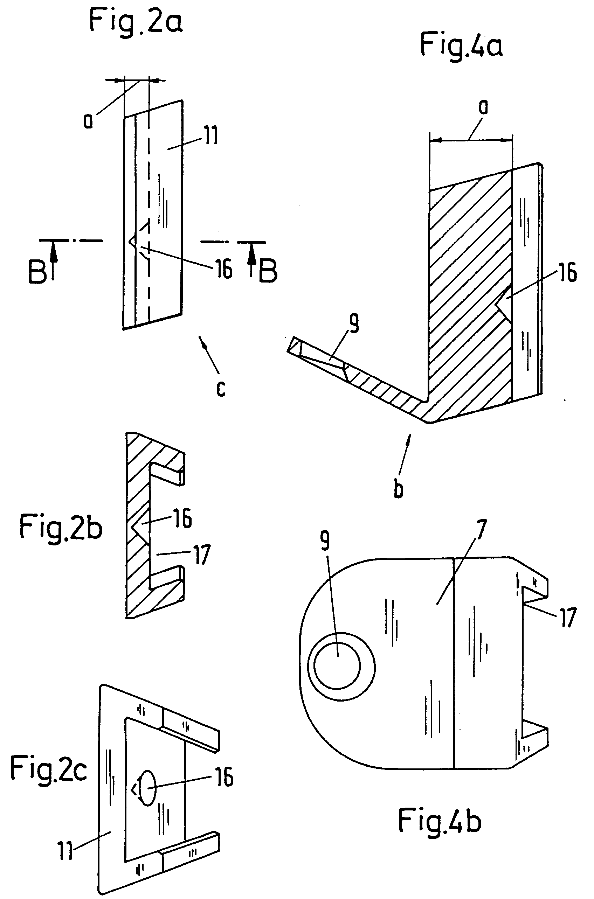

Für eine genaue Positionierung und Zentrierung des Stützkörpers 11 weist die Stiftschraube 13 (Fig. 6) eine kegelförmige Zentrierspitze 15 auf, die in eine daran angepasste, hohlkegelige Ausnehmung 16 (siehe z.B. Fig. 2b) eines Stützkörpers 11 eindringt.

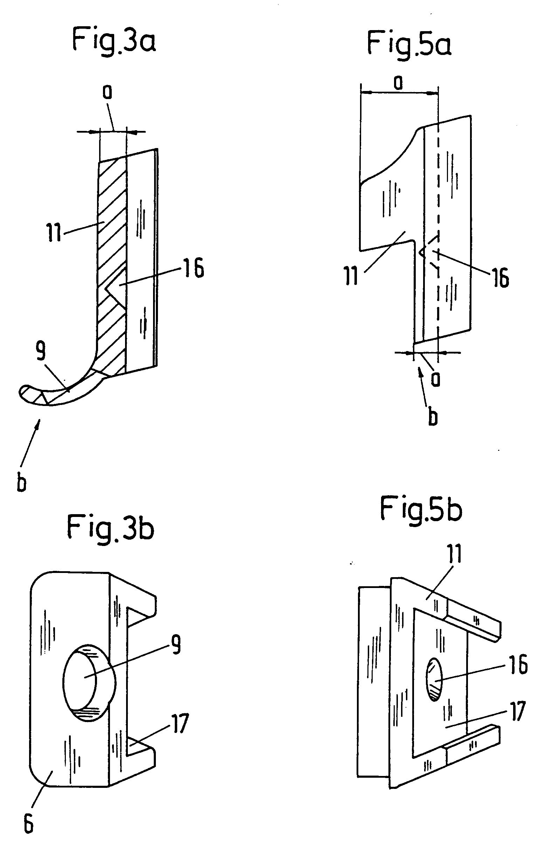

Wie bereits erwähnt kann jeder Hülse 1 eine ganze Anzahl Stützkörper 11 mit unterschiedlichen Abmessungen und Formen zugeordnet sein. Der einfachste Stützkörper 11 (Fig. 2) ist ein viereckiger Körper mit ebenfalls trapezförmigem Querschnitt; aus diesem Körper ist eine in Form und Abmessungen an den Bereich grösserer Wandstärke D oder Gleitschuh 3 der Hülse 1 mit kleinem toleranzenbedingtem Spiel angepasste schwalbenschwanzähnliche Ausnehmung 17 (Fig. 2b) herausgearbeitet, mit der der Stützkörper 11 den Gleitschuh umgreift. Die bei verschiedenen Stützkörpern 11 unterschiedliche Dicke in Radialrichtung - vergleiche z. Beispiel Fig. 3a und 4a - ist in allen Beispielen mit a bezeichnet.

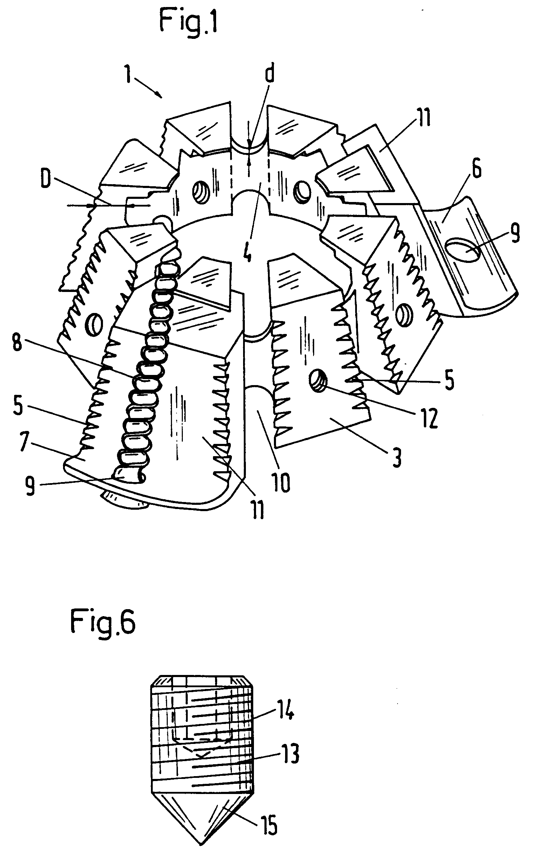

Bei dem Stützkörper nach Fig. 3 ist die einfache Form nach Fig. 2 an der basisseitigen Stirnfläche durch eine Lasche 6 ergänzt, die eine leicht gewölbte Form besitzt; in ihr ist eine Durchtrittsbohrung 9 vorgesehen, für den Durchtritt einer Knochenschraube 8 (Fig. 1).

Der Stützkörper 11 nach Fig. 4 unterscheidet sich von den beiden vorhergehenden zum einen durch seine grössere Dicke a und zum anderen dadurch, dass seine Lasche 7, die ebenfalls eine Bohrung 9 aufweist, eben ist und unter einem anderen Winkel an den Stützkörper 11 angesetzt ist als die Lasche 6.

Bei dem letzten gezeigten Beispiel eines Stützkörpers 11 ist seine Dicke a in radialer Richtung über die Mantelhöhe nicht konstant, sondern stufenförmig abgesetzt, wobei die grössere Dicke bei montiertem Stützkörper zur Polseite des Kegelstumpfes der Hülse 1 zu liegen kommt.

Wie die Mantelflächen der Bereiche 3 der Hülse 1 können auch die Mantelflächen der Stützkörper 11 mit einer Querverzahnung 5 (Fig. 1) versehen sein, um das An- und/oder Einwachsen von Knochengewebe zu fördern.

Selbstverständlich sind sowohl für die Stützkörper als auch für ihre Verbindung mit der Hülse und für ihre Fixierung und Zentrierung andere Formen und Konstruktionen möglich, wie z. Beispiel Doppellaschen oder andere, den individuellen Verhältnissen des Acetabulums angepasste Formen.

## Patentansprüche

1. Zweiteilige Hüftgelenkspfanne zur Verankerung im Becken-knochen, bestehend aus einer kegelstumpfförmigen Hülse (1) und einem die eigentliche Pfannenschale enthaltenden, im Passitz in den Innenhohlraum der Hülse (1) einpressbaren Einsatz, wobei die aus Metall gefertigte Hülse (1) in Umfangsrichtung Bereiche (3) grösserer Wandstärke (D) alternierend mit solchen (4) geringerer Wandstärke (d) aufweist, wobei ferner die Bereiche (4) geringerer Wandstärke (d) im Querschnitt Kreisringsektoren sind, während die Bereiche (3) grösserer Wandstärke (D) einen im wesentlichen trapezförmigen Querschnitt haben, der sich zum Pol hin konisch verjüngt, dadurch gekennzeichnet, dass die Bereiche (3) grösserer Wandstärke (D) als sich entlang des Mantel des Kegelstumpfes erstreckende Gleitschuhe für aussen aufschiebbare Stützkörper (11) ausgebildet sind, und dass jeder dieser Bereiche (3) mit Fixierungselementen (13) für diese Stützkörper (11) versehen ist.

2. Hüftgelenkspfanne nach Anspruch 1, dadurch gekennzeichnet, dass die Stützkörper (11) den Trapezquerschnitt der Bereiche (3) umgreifende, schwalbenschwanzähnliche Ausnehmungen (17) haben und vom Pol her auf die Gleitschuhe aufschiebbar sind.

3. Hüftgelenkspfanne nach Anspruch 1 oder 2, dadurch gekennzeichnet, dass Stützkörper (11) unterschiedlicher Dicke (a) vorgesehen sind.

4. Hüftgelenkspfanne nach einem der Ansprüche 1 bis 3, dadurch gekennzeichnet, dass mindestens ein Teil der Stützkörper (11) an ihrem der Basis des Kegelstumpfes zugewandten Ende mit nach aussen weisenden Laschen (6, 7) versehen sind.

5. Hüftgelenkspfanne nach Anspruch 4, dadurch gekennzeichnet, dass die Laschen (6, 7) mit Durchtrittsbohrungen (9) für Knochenschrauben (8) versehen sind.

6. Hüftgelenkspfanne nach einem der Ansprüche 1 bis 5, dadurch gekennzeichnet, dass die Dicke (a) der Stützkörper (11) von ihrem basisseitigen Ende aus in radialer Richtung stetig oder stufenförmig zu- oder abnimmt.

7. Hüftgelenkspfanne nach einem der Ansprüche 1 bis 6, dadurch gekennzeichnet, dass die Bereiche (3) grösserer Wandstärke (D) der Hülse (1) und/oder die Stützkörper (11) auf ihrer äusseren Mantelfläche mit einer Querverzahnung (5) versehen sind.

8. Hüftgelenkspfanne nach einem der Ansprüche 1 bis 7, dadurch gekennzeichnet, dass die Fixierungselemente (13) als die Bereiche (3) grösserer Wandstärke (D) durchsetzende Stiftschrauben (13) mit kegelförmiger Zentrierspitze (15) ausgebildet sind.

9. Hüftgelenkspfanne nach Anspruch 8, dadurch gekennzeichnet, dass die Stiftschrauben (13) in an ihre Zentrierspitze (15) angepasste, hohlkegelförmige Ausnehmungen (16) der Stützkörper (11) eingreifen.

## Claims

1. A two-part hipjoint socket for anchoring in the pelvic bone, consisting of a frustoconical shell (1) and an insert which contains the actual cup of the socket and may be pressed with a snug fit into the hollow inside the shell (1), the shell (1) being manufactured from metal and exhibiting regions (3) of greater wall thickness (D) which alternate in the circumferential direction with regions (4) having less wall thickness (d) and further the regions (4) having less thickness of wall (d) are in cross-section sectors of a circular ring, whilst the regions (3) of greater wall thickness (D) have a cross-section essentially trapezoidal which tapers in conically towards the pole, characterized in that the regions (3) of greater wall thickness (D) are made as guideshoes extending along the surface of the truncated cone for supporting bodies (11) which may be slipped on externally, and that each of these regions (3) is provided with fixing members (13) for these supporting bodies (11).

2. A hipjoint socket as in Claim 1, characterized in that the supporting bodies (11) have recesses (17) like dovetails which embrace the trapezoidal cross-section of the regions (3) and may be slipped onto the guideshoes from the pole.

3. A hipjoint socket as in Claim 1 or 2, characterized in that supporting bodies (11) are provided of different thicknesses (a).

4. A hipjoint socket as in one of the Claims 1 to 3, characterized in that at least some of the supporting bodies (11) are provided at their ends next the base of the truncated cone with flaps (6, 7) pointing outwards.

5. A hipjoint socket as in Claim 4, characterized in that the flaps (6, 7) are provided with holes (9) drilled in them for bone screws (8) to pass through.

6. A hipjoint socket as in one of the Claims 1 to 5, characterized in that the thickness (a) of the supporting bodies (11) in the radial direction increases or decreases steadily from their ends next the base or in the form of a step.

7. A hipjoint socket as in one of the Claims 1 to 6, characterized in that the regions (3) of the shell (1) having greater wall thickness (D) and/or the supporting bodies (11) are provided on their outer surfaces with sets of transverse teeth (5).

8. A hipjoint socket as in one of the Claims 1 to 7, characterized in that the fixing members (13) are made as setscrews (13) which have conical centreing tips (15) and pass through the regions (3) of greater wall thickness (D).

9. A hipjoint socket as in Claim 8, characterized in that the setscrews (13) engage in the supporting bodies (11) in hollow conical recesses (16) adapted to their centreing tips (15).

## Revendications

1. Acétabule en deux parties destinée à être ancrée dans l'os du bassin, constituée d'un manchon tronconique (1) et d'un insert contenant l'acétabule proprement dite, à enfoncer de manière ajustée dans la cavité intérieure du manchon (1), dans laquelle le manchon (1) réalisé en métal présente, dans la direction périphérique, des zones (3) de plus grande épaisseur de paroi (D) en alternance avec des zones (4) d'épaisseur de paroi (d) réduite, dans laquelle en outre les zones (4) d'épaisseur de paroi (d) réduite sont en coupe transversale des secteurs d'anneau de cercle, tandis que les zones (3) de plus grande épaisseur de paroi (D) ont une section transversale sensiblement trapézoïdale, qui se rétrécit coniquement vers le pôle, caractérisée en ce que les zones (3) de plus grande épaisseur de paroi (D) sont configurées en patins s'étendant le long de l'enveloppe du tronc de cône pour des corps d'appui (11) à enfiler de l'extérieur, et en ce que chacune de ces zones (3) est pourvue d'éléments de fixation (13) pour ces corps d'appui (11).

2. Acétabule selon la revendication 1, caractérisée en ce que les corps d'appui (11) ont des évidements (17) en queue d'aronde, entourant la section transversale trapézoïdale des zones (3) et peuvent être enfilés sur les patins, à partir du pôle.

3. Acétabule selon la revendication 1 ou 2, caractérisée en ce que des corps d'appui (11) d'épaisseur (a) différente sont prévus.

4. Acétabule selon l'une des revendications 1 à 3, caractérisée en ce qu'une partie au moins des corps d'appui (11) sont pourvus à leur extrémité tournée vers la base du tronc de cône, de pattes (6, 7) dirigées vers l'extérieur.

5. Acétabule selon la revendication 4, caractérisée en ce que les pattes (6, 7) sont pourvues de trous de passage (9) pour des vis à os (8).

6. Acétabule selon l'une des revendications 1 à 5, caractérisée en ce que l'épaisseur (a) des corps d'appui (11) augmente ou diminue constamment ou par degrés à partir de leur extrémité côté base, dans la direction radiale.

7. Acétabule selon l'une des revendications 1 à 6, caractérisée en ce que les zones (3) de plus grande épaisseur de paroi (D) du manchon (1) et/ou les corps d'appui (11) sont pourvues sur leur surface d'enveloppe extérieure d'une denture transversale (5).

8. Acétabule selon l'une des revendications 1 à 7, caractérisée en ce que les éléments de fixation (13) sont configurés en goujons filetés (13), traversant les zones (3) de plus grande épaisseur de paroi (D), avec pointe de centrage (15) conique.

9. Acétabule selon la revendication 8, caractérisée en ce que les goujons filetés (13) s'engagent dans des évidements (16) coniques et creux des corps d'appui (11), adaptés à leur pointe de centrage (15).
